# EUROPEAN PATENT SPECIFICATION

(11) **EP 2 629 723 B1**
(45) Date of publication and mention of the grant of the patent: **19.01.2022**
(21) Application number: 11834726.9
(22) Date of filing: 24.10.2011
(51) Int. Cl.: A61F 11/00, A61M 16/20, A61M 16/06

(54) **DEVICE FOR EQUALISATION OF THE PRESSURE IN THE MIDDLE EAR**
VORRICHTUNG ZUM AUSGLEICHEN DES DRUCKS IM MITTELOHR
DISPOSITIF D'ÉGALISATION DE PRESSION DANS L'OREILLE MOYENNE

(30) Priority: 22.10.2010 US 455539 P
(43) Date of publication of application: 28.08.2013
(73) Proprietor: Moniri Medical AB, 114 79 Stockholm (SE)
(72) Inventor: BIDARIAN MONIRI, Armin, 8500-367 Portimão (PT)
(74) Representative: Lourenço Martinho do Rosário, Ana Margarida
(86) International application number: PCT/SE2011/051260
(87) International publication number: WO 2012/053970

(56) References cited:
- WO-A2-2005/091871
- US-A- 3 330 272
- US-A- 3 749 083
- US-A- 4 896 666
- US-A- 5 419 762
- US-A- 5 950 631
- US-A- 5 950 631
- US-A1- 2002 078 952
- US-B1- 6 182 660

## Description

### FIELD OF THE INVENTION

The present invention relates to a device for obtaining an equalization of the negative pressure in the middle ear of a human being, a patient, having a face with a nose and a mouth. More specifically the invention relates to a device for pressure equalization in the middle ear requiring minimal collaboration from the patient. The device is suitable for treatment of both small children and adults.

### BACKGROUND OF THE INVENTION

Many people and in particular children suffer from a condition called secretory otitis media with effusion which leads to negative pressure and to the formation of liquid in the middle ear, causing hearing loss to those suffering therefrom. This condition normally develops after an upper airway infection, allergic reaction or sudden change of pressure when flying or diving. After a period of time of negative pressure the amount of mucous increases and with impaired drainage through the Eustachian tube, the formation of liquid in the middle ear is a consequence. This condition is called "secretory otitis media", "otitis media with effusion" or "glue ear" and leads to reduced hearing ability and also leads to an increased risk for other complications. The majority of patients suffering from secretory otitis media are children, and the anatomical conditions, i.e. the horizontal position of the Eustachian tube, is believed to contribute to the prevalence peaking in children younger than 4 years of age.

The negative pressure in the middle ear causing the secretory otitis media can in some patients be equalized by applying the "Valsalva maneuver". Many adults are able to perform this maneuver i.e. pressing the nostrils together, closing the mouth and by means of the diaphragm, increasing the pressure of the air in the mouth and nose cavity. The increased pressure so created is transplanted through the Eustachian tube, to the middle ear where it equalizes the negative pressure in the middle ear. This permits drainage of possible liquid in the middle ear, and reduces the symptoms of secretory otitis media.

Many children, however, cannot learn the Valsalva maneuver technique in order to facilitate the equalization of the negative pressure in the middle ear. Different devices have therefore been developed in order to help children to equalize the negative pressure in the middle ear, e.g. the so called Politzer balloon and various equipments aimed to facilitate the Valsalva maneuver, e.g. the Otovent balloon. The functioning of the Politzer balloon is to pump or press air into one nostril while closing the mouth and the other nostril and simultaneously swallowing, and force air into the Eustachian tube towards the middle ear. The Otovent balloon is adapted to be connected to one nostril and while holding the other nostril closed with e.g. a finger, the patient is instructed to close the mouth, swallow, and blow out through the nose to blow up the balloon to create a counter pressure for opening of the Eustachian tube and equilibration of the negative pressure in the middle ear. Both of these non-invasive devices have been of limited value to cure the secretory otitis media in children by equalizing the pressure in the middle ear. The main problems connected to these devices are probably due to difficulties of usage as they are complicated and also often unpleasant for small children to handle, resulting in low compliance.

For treating secretory otitis media, different methods have hitherto been used, namely adenoidectomy and the already mentioned Politzer and Otovent balloons. As far as we know however, the most common and efficient treatment for young patients suffering from secretory otitis media is paracentesis, usually in combination with the introduction of a ventilating tube in the tympanic membrane. Paracentesis is carried out most often during general anaesthesia. Due to the common complication of too early expulsion of the ventilation tube, this surgical procedure might need to be carried out repeatedly. Installation of a ventilation tube might also lead to a number of other complications such as chronic middle ear diseases and development of scars and/or permanent perforation of the tympanic membrane.

There are risks that are associated with general anaesthesia, like respiratory and circulatory failure, and there are already mentioned shortcomings of the methods and techniques presently available, related to poor medical results as well as to risks accompanying invasive methods. Therefore, demands have been raised for a non-invasive device for equalizing the negative pressure in the middle ear of patients, mainly children, who have problems to perform the Valsalva's maneuver and who have difficulties in using the Politzer or Otovent balloons and similar devices.

A new device is therefore disclosed for creating an excess pressure in the mouth and nose cavity in order to open up the Eustachian tube and force the air into the middle ear to equalize the negative pressure. The inventive device can be practiced on small children, and is very flexible in use as it can be operated by the child itself or it can be operated by a parent, with or without the active collaboration from the patient. The device here disclosed presents a documented patient compliance and is very well accepted among small children (as young as 1.5-3 years old) with no complications registered.

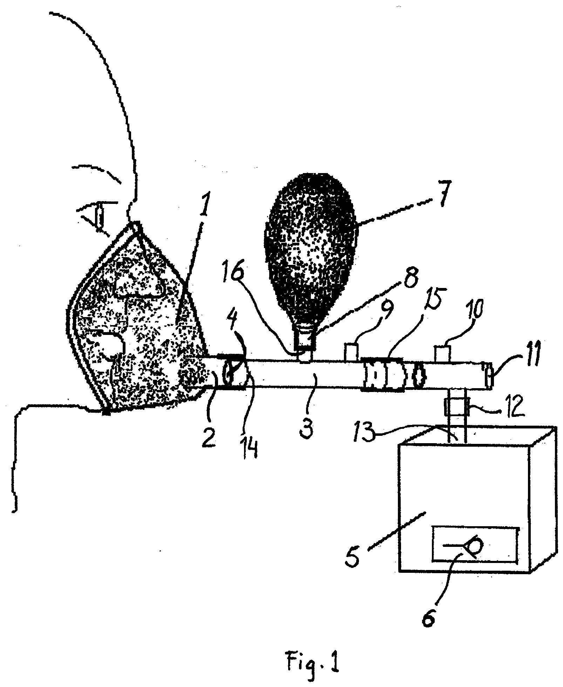

As shown in figure 1 the device comprises at least three components. A facemask is provided, adapted to cover at least nose and mouth of the patient. A manual or automatic air pump is provided, suitably covered with, or otherwise associated with, a teddy bear or similar toy figure like a puppet, for making the treatment more fun for the child. An air reservoir is provided, typically in the form of an elastic inflatable balloon, but alternative embodiments including other kinds of air reservoirs are also envisaged. All three components are connected to each other via a tube or similar structure having at least three openings adapted for the at least three main components, respectively. A pressure relief valve and/or an apparatus for pressure monitoring and/or an adjustable outlet valve also suitably are connected to the connecting tube or similar structure of the device.

Air pressure is created by the patient, by expiration into the facemask via mouth and/or nose, or by a practitioner or supervisor (e.g. doctor or parent), by activating the air pump, or is created by both patient and practitioner/supervisor/parent by essentially simultaneous expiration and pump activation, respectively. A sufficient rise in air pressure in the device makes the air reservoir expand. Ultimately this creates an increased pressure in the mouth and/or nose cavity and opens the Eustachian tube and forces air into the middle ear for equalization of the negative pressure and drainage of possible fluid.

As mentioned previously, various non-invasive equipments for equalizing pressure in the middle ear have been demonstrated in prior art. They are based on the idea of creating a positive air pressure in the nasal/mouth cavity by having the patient to blow a balloon with the mouth or with one or both nostrils (e.g. Otovent), wherein the positive air pressure of the balloon is transplanted to the nasal or mouth cavity, or are based on the Polizer technique, i.e. to force air into the nasal cavity by means of e.g. a balloon.

U.S. Patent No. 5,950,631 discloses a device for creating an increased pressure in the mouth cavity by inserting in the mouth of the patient a mouthpiece on which a balloon is mounted, and by instructing the patient to blow the balloon by expiration via the mouth while both nostrils are closed. The pressure created by the balloon is intended to be transplanted into the nasal cavity for entrance into the Eustachian tube and opening of the same. The flexibility in use is limited as the device is not adapted for nostril connection. This implies that the nostrils of the patient must be closed by hand or by the use of a nose clip. This makes the device according to this patent not very user-friendly compared to the device according to the present disclosure, as it does not allow the free choice of breathing and blowing through both nostrils and/or the mouth in the same way as the present disclosure does, and because the nose clip may hurt. Furthermore, there is no possibility for the practitioner or a parent to take part in the treatment as in the present disclosure which might exclude the youngest children that probably will have difficulties in understanding the procedure. Moreover, the effect in opening the Eustachian tube is limited as the passage from mouth cavity to Eustachian tube can be closed by closing the soft palate and letting all the air come out of the mouth instead of going towards the Eustachian tube. In conclusion, this device is limited in functionality and has a reduced compliance compared to the present disclosure.

EP0504124, U.S. Patent No. 3,749,083 and U.S. Patent No. 4,817,626 all have a very similar appearance and functionality. The devices are composed of a tube having one end adapted to fit one of the patient's nostrils, and the other end provided with a balloon. The devices have the nostril end of the tube adjacent to the patient's nostril and when the balloon is inflated, the pressure created by the inflated balloon is intended to be transplanted into the nasal cavity for entrance into the Eustachian tube and opening of the same. Many adults and most children experience difficulties understanding and learning how to use these devices. Even if young patients understand the instruction of usage, there are often problems related to proper positioning against the nose - if the device is too heavily pressed against the nose it actually tends to block the nostril opening, and also hurts, and if it is too loosely pressed against the nose it tends to create leakage. Many patients break off the treatment due to unpleasantness and pain and discomfort associated with the treatment. The flexibility in use is limited as the devices are not adapted for mouth connection. This implies that the patient's mouth and one of the nostrils must be closed making these devices not very user-friendly compared to the device according to the present disclosure, as they do not allow the free choice of breathing and blowing through both nostrils and/or the mouth in the same way as the present disclosure does. This problem is not solved simply by providing a Y-shaped tube with two nostril openings as is proposed in EP0504124, which makes the adaptation and usage even more complicated for the young patient. The free choice of breathing is still very limited, and furthermore, having the nostrils occupied by one or two tube-end nostril plugs is not a very pleasant feeling for most persons, definitely not children. Furthermore, there is no possibility for the practitioner or parent to take part in the treatment as in the present disclosure, which excludes the youngest users who cannot understand the given instructions. Having a pump increasing the pressure as in the present disclosure is specifically important when treating children with limited understanding of the treatment and this enables success in children as young as 1.5 years of age. It has been previously reported that the device according to EP0504124 has not been proven to give satisfactory results on patients younger than 5 years old, which is in contrast to the present disclosure by which it is possible to reach good results on patients from 1.5 years old and older. In conclusion this prior art device is limited in functionality and has a reduced compliance compared to the present disclosure.

U.S. Patent Nos. 4,749,377; 5,419,762 and 2006/0272650 disclose various types of equipment for equalizing pressure in the middle ear, all of them based on the idea of creating a positive air pressure in the nose/mouth cavity by exposing the airways to a positive air pressure created by a device delivering compressed air via an electrical air pump via a single nostril plug. The provision of a single nostril plug creates similar disadvantages as the previously mentioned devices with a balloon and a tube end for nostril connection. A difference from those previously mentioned devices, however, is that the practitioner controls the flow rate and air pressure by regulating the equipment. On the other hand, the patient has a very limited control over the situation, which usually ends up in unpleasant experiences and low compliance, especially when it comes to treatment of children. These devices can be referred to as relying on the same treatment regime as the Polizer balloon, which treatment is well known to be considered by younger patients to be very unpleasant, which in turn results in a poor compliance.

In US Patent 3330272 a Resuscitator for the newly born patient is described. The device shown in the application description and on its drawings consists basically in a body, means to attach to the body a resilient bulb and a ventilating device. A passage in the body is connecting the bulb cavity to the ventilating device. A port from the passage to the atmosphere is arranged and a one-way valve is controlling the flow through the port and is closed by pressure within the passage. A pressure-relief orifice is connecting the passage to the atmosphere.

As indicated in the patent title, the device is designed to help newly born babies to start breathing by pushing air into the lungs of the baby through the mouth and airways leading to the lungs. For this purpose, the device comprises a resilient bulb, a rubber bulb. The rubber bulb 56 functions as a pump which is designed to be deflated by hand in order to push air into the lungs of the baby. When the hand grip is removed, the bulb strives to regain its initial shape which cannot then be exceeded significantly. The breathing of the baby can assist in the bulb regaining its initial, given shape, but then excess of air has the possibility to pass to the surrounding atmosphere through the pressure-relief orifice.

The present disclosure is better, and has indeed demonstrated unexpected and superior results, compared to the technical solutions of the prior art, and this is for several reasons. Firstly, the patient does not need to precisely follow a specific procedure of breathing out and simultaneously swallow to get the desired effect. Secondly, the patient is free to breathe out through the mouth or the nose or through both nose and mouth, and this has been shown to be very important for compliance and for the treatment efficacy and the comfort of usage. Prior art devices are restricted to either mouth or nose application, or even to one single nostril, having the other nostril closed by e.g. a finger. Thirdly, the supervisor, which preferably is a parent, can create the pressure in the system (device and patient airways) by activating the air pump whenever the patient is exhaling or breathing actively or not. So the parent can, but he/she does not have to, help the patient fill the air reservoir with air to get an increased pressure in the system, which increases the flexibility of usage and adaptation to the particular patient. In a typical embodiment it is possible to blow the balloon, to have it filled and exert a counter pressure, or creating the counter pressure using the pump without the active participation of the patient. It has unexpectedly been found that an unaffected teamwork or collaboration between the young patient and the supervisor/parent is developed, which is considered a joyful game for the patient, by using the disclosed device, which obviously is part of the successful treatment results demonstrated.

Thus it is an object to provide an improved non-invasive device for pressure equalization of the middle ear of patients, mainly children, suffering from secretory otitis media.

### SUMMARY OF THE INVENTION

The invention herein is a device for obtaining an equalization of a negative pressure in the middle ear. The device comprises a facemask, an air pump and an air reservoir, all of them connected to a connecting tube. An increased air pressure is created by expiration into the facemask and/or by activating the air pump. A sufficient rise in air pressure in the device makes the air reservoir expand, and this creates increased pressure in the mouth/nose cavity and opens the Eustachian tube and forces air into the middle ear for equalization of the negative pressure.

More specifically, device comprises a facemask, which facemask is adapted to tightly cover at least the nose and mouth, thereby creating an air chamber between the face and the facemask, and which facemask has a facemask air channel which allows air flowing in or out of the chamber, an air source, having the capacity to deliver an increased air pressure compared to the atmospheric air pressure through an air source outlet, an air reservoir, having a reservoir air channel which allows air flowing in or out of the reservoir, which air reservoir is designed to define and maintain an air pressure exceeding the atmosphere pressure and work like a pressure regulator when loaded, and a tube structure, having at least three openings adapted for connecting the facemask air channel, the air source outlet and the reservoir air channel to the tube structure interior. The air reservoir 7 is an elastic inflatable balloon, or a variable volume chamber which is e.g. spring or gravity loaded.

The air reservoir in one embodiment can be designed to maintain a counter pressure of 200 - 800 daPa (20 - 80 mBar) when loaded.

Yet another embodiment comprises a security pressure relief valve for pressure monitoring the tube structure interior, connected to the tube structure.

Still another embodiment comprises an adjustable relief valve, which is connected to the tube structure in order to control the maximum pressure of the tube structure interior. The adjustable relief valve can be adjustable to release pressure in the range from 0 to 1000 daPa (0 to 100 mBar)

A further embodiment comprises a check valve, which is connected between the air source and the tube structure, allowing air flow into the tube structure only.

In yet another embodiment, the check valve is arranged to cooperate with the adjustable relief valve so that when the check valve is open the release valve is closed.

According to the invention, the tube structure can be designed to comprise one or several interconnected tube shaped components, coupled to each other.

In one embodiment of the invention an air source in the form of a powered air pump is provided, which suitably has means for not permitting air flow in the opposite pump direction. In another embodiment the air source is a manually operated bellow pump.

The invention also, in another embodiment, comprises a facemask which is removable from the connecting tube 3 in order to allow a choice of facemask of a size well suited for the human being in question.

In one embodiment, suitable for small children, the facemask is provided with a piece resembling a baby pacifier or a rubber nipple. The piece can then be replaceable and optional for installation in the facemask. In another embodiment suitable for small children, the air reservoir and/or the air source is hidden in a funny figure in the shape of a puppet or a cuddle toy. Also, in yet another embodiment the device comprises means for providing optical or visual feedback to the patient when the air reservoir is loaded.

Other objects and advantages of the present invention will become obvious to the reader and it is intended that these objects and advantages are within the scope of the present invention.

### BRIEF DESCRIPTION OF THE DRAWINGS

Figure 1 is a perspective view showing a representative embodiment of the present invention mounted over the nose and mouth of a user.
Figure 2 is a perspective view showing an alternative embodiment of the present invention mounted over the nose and mouth of a user.
Figure 3 is a collection of side view drawings showing various configurations of the present invention.
Figure 4 is a perspective/side view drawing showing the present invention in a preferred embodiment comprising a teddy bear puppet in which an air pump is inserted.
Figure 5 is a perspective/side view drawing of a detail of the device according to the present invention.

### DETAILED DESCRIPTION OF THE INVENTION AND

### PREFERRED EMBODIMENTS THEREOF

The device here disclosed comprises at least three main components connected to a tube or similar structure composed of one or several interconnected tube shaped components, and hereafter referred to as the connecting tube.

Referring to figure 1, a facemask 1 is provided as a face interface, adapted to provide an essentially tight-fitting cover for at least both nose and mouth of the patient. The facemask is provided with an opening 2 for connection to a connecting tube or tube structure 3, wherein the opening 2 of the facemask communicates as a mouth/nose-interface creating an open air channel through the facemask 1. The facemask 1 preferably is removable from the connecting tube 3 in order to allow a facemask 1 of a size well suited for the patient in question to be provided, e.g. a small size if the patient is a child, or a larger size for adults. Any size of the facemask 1 is envisaged in this context. The facemask 1 can be disposable or not, and can be provided with a filter 4 for cleaning the breathing air before entering the device. The facemask 1 does not have to be a conventional facemask, but could be any kind of structure that can provide an essentially tight-fitting cover over the nose and mouth of a patient, and that is able to conduct air between the mouth/nasal cavities and the other parts of the device. The part of the facemask 1 coming into contact with the patient's skin can be made of silicone, gel, leather, rubber, plastic, e.g. as an air-filled, gel-filled or water-filled pillow, or can be made of any material that fits the facial contours in a sealing manner and is acceptable in terms of human health. The rest of the facemask 1 can be made of similar material, however typically a more rigid structure.

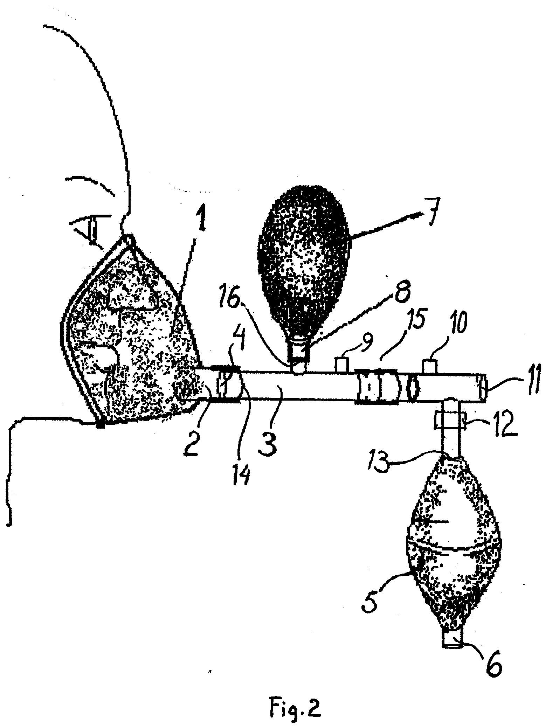

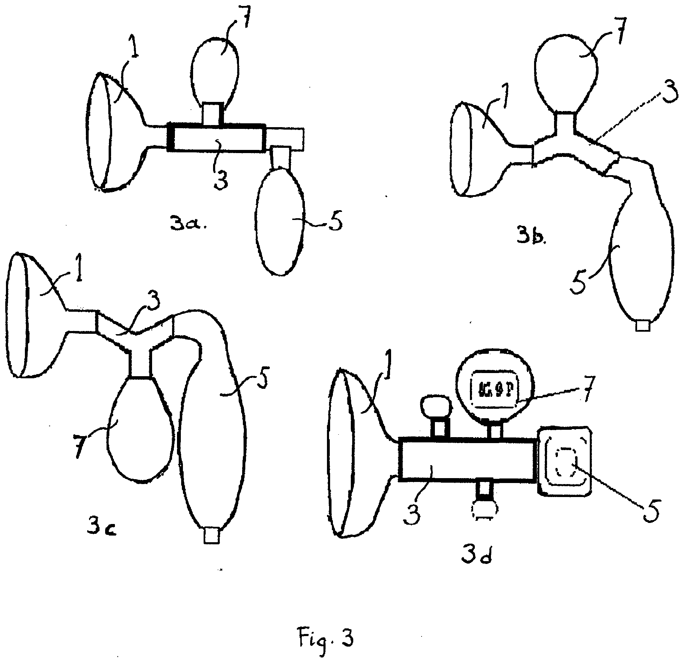

An air source, such as an air pump 5 or air compressor or other apparatus having the capacity to deliver an increased air pressure compared to the atmospheric air pressure, is provided. The air pump 5 or air compressor preferably is intermittently activated, or is provided with an internal outlet regulator, to provide an increased air pressure, i.e. compressed air, discontinuously or continuously. The air pump 5 could be manual (hand-powered or foot-powered; figures 2, 3a-3c) or could be an automatic or semi-automatic powered electromechanical or pneumatic or hydraulic pump (figures 1 and 3d) or could be a pump based on a combination of the foregoing technologies. The air pump 5 does not permit air flow in the opposite direction (back-flow), regardless of being activated or not activated. Examples of manual pumps are bellow pumps, compressible balloons or balls, similar to mattress pumps, or any kind of displacement pump or reciprocating pump, like for example piston pumps. The air pump 5 or air compressor is provided with an air outlet 13 connected to the connecting tube 3. In an embodiment the air pump 5 is removable, allowing pumps/compressors of different types, sizes and capacities to be provided as desired.

Referring to figure 2, in the embodiment comprising a manual air pump 5, a check valve 6 can be provided for regulation of the air flow between the pump 5 and the outside atmosphere, allowing air flow into the pump 5 when the internal air pressure is lower than the surrounding atmosphere, not allowing air flow out of the pump 5 when the internal air pressure is higher than the surrounding atmosphere. The provision of the check valve 6 enables successive pumping of air from the pump into the connecting tube.

Referring to figure 1 and 2, an air reservoir 7 with an opening 8 adapted for connection to the connecting tube 3 is provided. Preferably, the air reservoir 7 is an inflatable balloon, but it could also be a variable volume chamber which is e.g. spring or gravity loaded. The air reservoir 7 is provided to provide a defined air pressure resistance when loaded in order to provide a counter pressure optimal for pressure equalization of the middle ear without causing any hazard to the tympanic membrane or the lungs. Other technical solutions based on air reservoirs in combination with digital pressure control and pressure monitoring and feedback are also envisaged. The air reservoir 7 is preferably removable, allowing air reservoirs of various types and of various air pressure resistances to be provided, and so that used and non-functioning air reservoirs can be replaced by new ones as demanded. In the case of the air reservoir 7 being a balloon, this is suitable disposable.

Each one of the at least three main components, 1, 5 and 7 in figures 1 and 2 are connected to the connecting tube 3 which has at least three openings 14, 15, 16 receiving the corresponding openings of the at least three main components, respectively. The components are communicating via the connecting tube 3. Air pressure can be created by the patient breathing or blowing into the facemask 1 via mouth and/or nose, or can be created by activating the air pump 5 or by a combination of the two procedures. Air pressure so created propagates along the inside of the connecting tube 3 to the different components and if it reaches a certain level it makes the air reservoir 7 to expand and/or stay expanded. It also creates an increased pressure in the mouth and nose cavity in order to open the Eustachian tube and force the air in the middle ear to equalize the negative pressure therein and drain any possible fluid.

Referring to figure 2 an apparatus for pressure monitoring 9 and/or a security pressure relief valve 10 and/or an outlet valve 11 with adjustable opening pressure may also be provided to allow monitoring the pressure, and avoiding the creation of a pressure that can cause damages to the patient's ears or other organs.

The connecting tube 3 shown in e.g. figure 2 is preferably made of plastic or metal of sufficient rigidity to provide air-tight connections to the various components connected to it, and for easy handling of the device. The connecting tube may also have details made of other materials like rubber, metal and glass and parts of it may be more flexible to make it adjustable if so desired. The connecting tube 3 can be made entirely as one unit, or can be composed of several parts connected to each other, e.g. parts of tubes, joints and gaskets.

Again referring to the figures 1 and 2, an embodiment of the present disclosure comprises a face mask 1 as an interface adapted to essentially tight-fittingly cover at least the nose and the mouth of a patient, in the figures represented by a facemask 1 with minimum leakage of air during respiration with the facemask properly mounted on the face of a patient. The facemask is connected to the first opening 14 of connecting tube 3 or similar structure. Furthermore the embodiment comprises an air pump 5. The air pump 5 is connected to the second opening 15 of the connecting tube 3. Furthermore it comprises an air reservoir 7, in the figure represented by an inflatable balloon, for controlling the air pressure in the device, for providing compliance feedback to the patient, regulating the pressure in the system, and finally for providing an expandable air reservoir function. The air reservoir 7 is mounted on the connecting tube 3, and connected to the third opening 16 of the connecting tube.

In the present disclosure, the air reservoir 7 is connected to the third opening 16 of the connecting tube and the air pump 5 is connected to the second opening 15 of the connecting tube 3, as shown in figures 1 and 2. Various spatial relationships between the various components of the device of this disclosure are demonstrated in figures 3a, 3b, and 3c. However other configurations not particularly represented by these figures are also envisaged.

Preferably, an adjustable relief valve is provided such as outlet valve 11 to control the maximum pressure in the device. The relief valve preferably is adjustable to release pressure in the range from 0 and 1000 daPa (0 and 100 mBar).

The position of the relief valve is not fixed, but it can be positioned as desired, e.g. as indicated in figures 1 and 2.

In another embodiment, with reference to figures 1 and 2, a check valve 12 is provided between the air pump 5 and the connecting tube 3, which check valve 12 regulates the air flow between the air pump 5 and the connecting tube 3 and the surrounding atmosphere. The adjustable valve 11 is designed to mainly regulate the maximum pressure in the tube 3 but also to cooperate with the check valve 12 in the following way. The check valve 12 allows air flow from the air pump towards the balloon and facemask 1 via the connecting tube 3 when the air pressure of the air pump exceeds the air pressure in the connecting tube 3, e.g. during activation of the air pump, and at inspiration, while sealing off the adjustable valve 11. Thus check valve 12 allows airflow only towards the patient whenever the pump is activated and allows airflow from the patient out through the adjustable valve 11 whenever the pump is not activated. The check valve 12 does not allow any air back flow through the pump to the atmosphere at any time. The expiratory air is released from the device to the outside atmosphere via the adjustable outlet valve 11 against a certain pressure resistance that can be set within a range from 0 to 1000 daPa (0 to 100 mBar)

This embodiment allows the patient to breathe normally by both inspiration and expiration through the facemask with no discomfort whenever the resistance is set on 0 or close to 0 and the air pump (5) is not activated. When the pump is activated the check valve 12 opens up for air flow directed towards the patient and seals off the adjustable outlet valve 11. At inspiration when the pump is not activated the air enters through check valve 6 and passes the pump 5 and reaches the patient through the connecting tube 3 and the mask 1. The expiratory air passes out through the system and can only pass to the outside through the adjustable outlet valve 11. Only when the air pump is activated (increased pressure) the patient's expiration is trapped within the device and, together with the air from the activated air pump 5, contributes to inflating the air reservoir 7 resulting in an increased internal air pressure in the device and in the mouth/nose cavity. The resistance of the air flow out of the system through outlet valve 11 can be adjusted by altering the size of the opening and is gradually raised to reach the desired level with the acceptance of the patient without causing any discomfort.

In an alternative embodiment of the device there is no adjustable valve provided. In this embodiment the device comes into function by the internal air pressure rising as soon as the patient breathes or blows by expiration into the facemask 1 irrespective of having the air pump 5 activated or not. In this embodiment the air pump 5 is activated only when desired, e.g. when in use on small children with the lack of sufficient collaboration. An advantage of this embodiment is that no coordination between the patient's expiration and the activation of the air pump 5 is needed. Also, in this embodiment it is easier for a child to handle the entire device on his/her own, without the assistance of a parent or other supervisor.

Preferably, the embodiment provided with an adjustable outlet valve 11 and a check valve 12 is used initially, to get the patient familiar with the device. The pressure gradient is gradually raised by adjustment of the outlet valve 11 from 0 towards the desired pressure which should be higher than the pressure needed to inflate the balloon. The resistance in the adjustable outlet valve 11 can be set between 0 and 1000 daPa (0 and 100 mBar)

The embodiment without an adjustable valve 11 could be used at a later stage of treatment, e.g. under supervision of a parent to the patient or without any supervision.

The arrangement with an adjustable outlet valve 11 according to the foregoing description allows the patient to breathe freely without substantial resistance when the device is tight-fittingly mounted at the mouth and nose of the patient, the resistance on the adjustable outlet valve 11 is 0 or close to 0 and the compression bag is not compressed. This minimizes the sense of discomfort in wearing the device and is therefore an advantage compared to prior art devices.

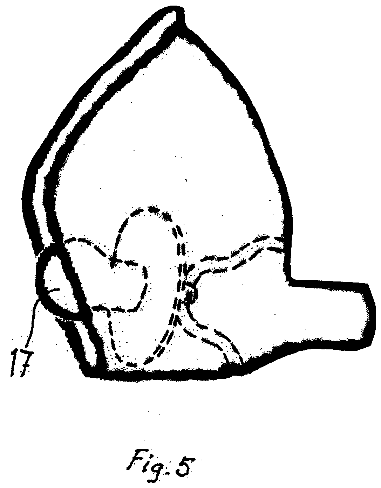

In an optional embodiment the facemask 1 is provided with a component 17 resembling a baby pacifier or a rubber nipple provided in elastic materials such as rubber or silicone with or without a taste or flavour upon contact with liquid (patients saliva) or suction. According to figure 5, this pacifier like structure would be fixated inside the facemask 1 with a mouldable or elastic mechanism to permit limited movement for adjustment to the patient's facial configuration and to be comfortably adapted in the mouth when the facemask is in position. This optional embodiment of the facemask would inspire the child to want to play with the device even more improving the compliance, assuring a closed mouth while performing the treatment leading to transplantation of the raised pressure within the nasal cavity and towards the Eustachian tube and finally stimulates salivation, suction and swallowing which is known to reduce the pressure needed to open the Eustachian tube, leading to improved efficiency as well. The component 17 could be replaceable and optional for installation in the facemask or could be stationary fixed.

The device according to the present disclosure is preferably intended for treatment of children with negative pressure in the middle ear, causing secretory otitis media. Adults can also successfully be treated with the device of the present invention. The device is advantageously used by children as well as adults in connection with diving and flying and other activities that could induce disadvantageous pressure gradients over the tympanic membrane. Treatment using the device can be performed at any stage of dysfunction and any degree of symptoms related to pressure gradients over the tympanic membrane. The treatment can also be prophylactic.

In the embodiment, provided with an adjustable valve 11, the device functions so that the interface, such as facemask 1, adapted to cover the mouth and nose is placed over the mouth and nose of the patient so that it allows little or no leakage of air during respiration. The patient breathes through the device freely with the air flowing in, coming into the system through check valve 6, passing the air pump 5 and the check valve 12. The outflow of the expiratory air is provided through outlet valve 11 when the resistance is set lower than the counter pressure of the air reservoir 7. The adjustable outlet valve 11 set on zero gives essentially no expiration resistance from the valve. Whenever the air pump 5 is activated upon expiration or apnea (no breathing) a positive air pressure will be created within the device and this air pressure will propagate into the airways increasing the air pressure in the mouth and nose cavity, making the air reservoir 7 larger (in the case it is a balloon) creating a counterpressure and leading to the opening of the Eustachian tube and equalization of the negative pressure in the middle ear. By raising the resistance in the adjustable valve 11 it is possible to gradually adapt the patient to expire towards a resistance and to finally be able to blow up the air reservoir 7 (i.e. increase the air pressure) by her-/himself i.e. when the resistance is higher than the pressure required to expand the air reservoir 7.

In the embodiment not provided with an adjustable valve 11, the device functions so that the interface, such a face mask 1, adapted to cover the mouth and nose is placed over the mouth and nose of the patient so that it allows little or no leakage of air during respiration. Whenever the patient blows, or at expiration into the device, and/or when the air pump 5 is activated, a positive air pressure will be created within the device and this air pressure will propagate into the airways increasing the air pressure in the mouth and nose cavity, due to the air reservoir 7 becoming larger (if it is a balloon) or increasing the pressure by other means, creating a counterpressure and leading to the opening of the Eustachian tube and equalization of the negative pressure in the middle ear.

The function of the air reservoir 7 is to maintain a positive air pressure in the device and in the patient's mouth and nose cavity for a while, typically for about 1 to 2 seconds, in a preferred embodiment for 2 to 5 seconds, to work like a pressure regulator not permitting dangerously high pressures in the system, and to give feedback on the compliance. The compliance feedback can be based on the air reservoir being a balloon 7 that expands as the pressure increases upon expiration, or can be based on indirect digital or analog signals indicating an increased pressure in the system, like sound or light or colors or a combination of the these, typically a visual and/or audible signal on a display and/or loudspeaker. In a preferred embodiment, the air reservoir 7, e.g. a balloon or other pneumatic equipment provided with a pressure loaded chamber, or a pressure gauge, can have a funny figure or the like on it or on a cover put on it or simply in association to it, to provide compliance feedback more adapted to children as the air pressure in the air reservoir is increased.

The air reservoir 7 also helps in adjusting the desired pressure. Using a balloon for this regulator purpose, it must have an elasticity which gives an inflating resistance in order to achieve a therapeutic effect, whereby the counterpressure preferably is 200 up to 800 daPa (20 up to 80 mBar). The balloon must be of such elasticity to provide a reservoir to maintain the desired pressure in the airways and keep the Eustachian tube open for at least 1-5 seconds, allowing equalization of the negative pressure as well as drainage of any possible fluid in the middle ear.

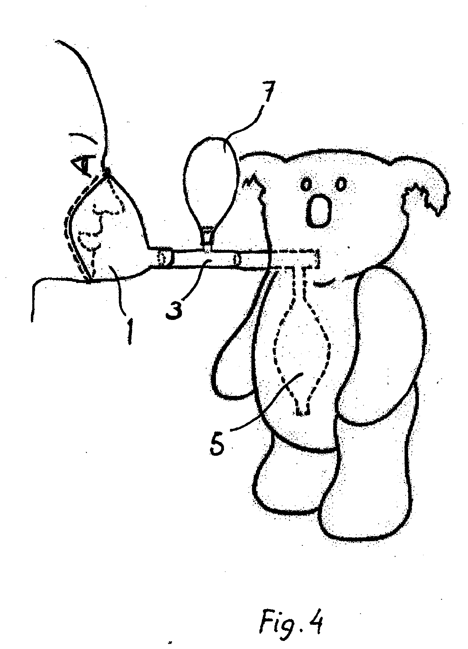

In a preferred embodiment the air pump 5 is provided with a funny figure on it or on a cover put on it, preferably a teddy bear or similar cuddly toy in the form of e.g. a puppet mounted on the outer surface of the air pump or in association with the air pump, and with the air tube 3, mounted for example through its front (figures 4). In an alternative embodiment both air pump 5 and air reservoir 7 are covered by the funny figure (not shown). The figure is preferably made of a material that the patient finds attractive and pleasant, e.g. textile, teddy, nonwoven fabric, fur, rubber, plastic, silicone or a combination of these and other materials. The supervisor/parent typically "helps" the child patient increase the air pressure in the device to get compliance feedback (e.g. blowing the balloon or inducing the compliance feedback system described above to provide a feedback signal). This is done by squeezing the teddybear or similar figure, and thereby simultaneously force air from the pump (figure 4) into the tube, air reservoir and into the patient's mouth/nose cavity. The patient's response to the "help" is typically to blow the balloon by expiration, and this is part of the treatment. In an alternative way of practice, the young patient "helps" the teddy bear puppet to blow the balloon up (the air reservoir) to get positive compliance feedback, which leads to an increased pressure in the nose/mouth cavity, resulting in a successful treatment. There is often an unaffected teamwork play or collaboration developing between the young patient and the practitioner/supervisor/parent as the device here disclosed is used, which obviously is part of the successful treatment results.

By gradually increasing the resistance in the adjustable outlet valve 11 the child learns to breathe against a constant pressure which at a certain level will increase the pressure in the upper airways enough to blow up the balloon. After a short time of training to get familiar with the device, many children will prefer to have the embodiment with no adjustable valve 11 to be able to blow up the balloon by themselves even without the help of the parent.

The device functions by increasing the air pressure in it whenever the patient expires in it or when he/she expires or holds her/his breath in combination with activation of the air pump. Therefore it is possible to use it with little collaboration from the patient and even on small children with difficulties in understanding and following instructions.

The optional security device comprising an apparatus for security pressure relief valve 10 can be set on a desired pressure. This security pressure relief valve is opened whenever the pressure in the system exceeds a certain level e.g. 200 - 800 daPa (20 - 80 mBar).

In summary, the device according to the present disclosure relies on a combination therapy based on both the Valsalva and Polizer techniques. This combination has been proven to give surprisingly good therapeutic results of curing secretory otitis media. The unexpected results are much better than the sole additive effect of Polizer in combination with Valsalva. This will be described more in detail in the following examples. Moreover, in a preferred embodiment the present disclosure relies on the psychological dimension of treatment of children by providing feedback compliance based on the increased air pressure, and by a playmate associated with air pump. These aspects of the disclosure are important for a positive relation to be established between the young patient and the device and the parent/supervisor or practitioner when he is present, and thereby increases the compliance. In conclusion, this invention is more user-friendly and reaches much better results compared to the already known technical solutions of treatment of secretory otitis media.

### EXAMPLE 1

A pilot study conducted on four subjects with serous otitis media demonstrated 100% compliance and immediate improvement of the pressure of the middle ear after 20 minutes of usage. The subjects, aged 2-7 years, were collected from the waiting list for insertion of ventilation tubes in general anaesthesia at the hospital. The criteria for inclusion for surgery are bilateral otitis media with effusion for more than 6 months assessed by tympanometric and clinical evaluation.

This study was small but of value to demonstrate that the device was able to produce the desired effect and reduced the negative pressure in the middle ear of the test subjects.

### EXAMPLE 2

A pilot study conducted on patients aged 1-6 years (from the waiting list for insertion of ventilation tubes at the hospital) showed improved tympanometric results in 9 of 11 of the test subjects with otitis media with effusion treated 2 times a day by the device of the present invention for equalization of the middle ear pressure during 4 weeks.

The middle ear pressure was measured at inclusion and after 2 and 4 weeks of treatment with the device.

Results:
- One patient refused to use the device.
- One patient has not experienced desired clinical effect of the treatment.
- All the other subjects have objectively improved middle ear pressure confirmed by tympanograms.
- No adverse effects were reported.
- The device was well tolerated by the children. They found it as interesting as a toy, and therefore accepted the treatment.
- The youngest subject, 1.5 years of age, had been suffering from serous otitis media for about 6 months. This had limited his hearing, the language development and his social skills. The boy used the device as prescribed and improved his middle ear pressure to normal after 4 weeks of treatment. The parents noted a substantial improvement in his hearing and the verbal development. Along with the treatment he became able to pronounce new words and became much more active and participating in the family.
- The cured patients were excluded from the waiting list for operation.
- The patients are followed up in the out-patient clinic and have so far maintained normal hearing and middle ear pressure.

### EXAMPLE 3

An ongoing study conducted at the Sahlgrenska University Hospital in Gothenburg Sweden shows promising results for the invented device regarding the middle ear pressure and hearing in young children. The patients were chosen from the waiting list of the hospital for operation of serous otitis media and only subjects with middle ear pressure < -400 were included. The included subjects had been suffering from hearing loss due to the negative pressure and serous otitis media during the past 6 months and were about to be operated under general anesthesia for insertion of ventilation tubes.

The patients were randomized into a study and a control group and the audiologist and the treating doctors were blinded for the treatment given. The study group received treatment twice a day with the invented device. Both the study and the control group underwent hearing and tympanometric tests at inclusion and after 2 weeks.

The middle ear pressure is measured in dekapascal (daPa) and the closer the pressure is to 0 the better the movement of the tympanic membrane and the better the hearing. In young children, it is normal to find negative pressures in the middle ear and a pressure from - 200 to 0 is considered normal in small children.

The hearing level is measured in decibel (dB) and is registered by the patient distinguishing the tones presented. The lower the level of sound that the patient distinguishes the better is the hearing.

The subjects were instructed to use the device twice a day to make 15 balloons in the morning and 15 in the evening. The patients' parents kept a diary about usage, any adverse effects or infections.

Results:
- No adverse effects were reported and on the contrary the test subjects found the treatment amusing.
- The infection rate was the same as in the control group
- 7 of the 9 patients in the treated group improved their hearing and middle ear pressure compared with 1 of 9 in the control group.
- The 2 patients in the treatment group with no therapeutic effect did not fulfill the treatment as described.
- The average middle ear pressure in the treated group improved from -400 daPa to -152 da Pa (table 1) while the pressure for the subjects in the control group improved slightly from -400 da Pa to -370 daPa. (table 2)
- The average hearing level in the treated group improved from 21.6 dB to 13 dB (table 1) while the hearing level in the control group altered slightly from 23 dB to 21.6 dB. (table 2)

**Table 1: shows the results in the treated group**

| Treated Group | | At Inclusion | | After 2 Weeks | |
|---|---|---|---|---|---|
| Nr | Age (year ) | Middle Ear Pressure (da Pa) | Average Hearing Level (dB) | Middle ear Pressure (da Pa) | Average Hearing Level (dB) |
| 1 | 3 | <-400 | 21 | -155 | 13 |
| 2 | 4 | <-400 | 24 | -175 | 13 |
| 3 | 2 | <-400 | 19 | -55 | 11 |
| 4 | 2 | <-400 | 18 | -105 | 10 |
| 5 | 3 | <-400 | 24 | -125 | 13 |
| 6 | 2 | <-400 | 19 | -205 | 8 |
| 7 | 4 | <-400 | 26 | -75 | 9 |
| 8 | 3 | <-400 | 23 | <-400 | 20 |
| 9 | 4 | <-400 | 20 | -75 | 20 |
| **Mean** | **3** | **-400** | **21,6** | **-152** | **13** |

**Table 2: shows the results in the control group**

| Control Group | | At Inclusion | | After 2 Weeks | |
|---|---|---|---|---|---|
| Nr | Age (year ) | Middle Ear Pressure (da Pa) | Average Hearing Level (dB) | Middle ear Pressure (da Pa) | Average Hearing Level (dB) |
| 1 | 2 | <-400 | 23 | <-400 | 23 |
| 2 | 3 | <-400 | 26 | <-400 | 24 |
| 3 | 3 | <-400 | 21 | <-400 | 19 |
| 4 | 3 | <-400 | 24 | <-400 | 26 |
| 5 | 4 | <-400 | 19 | <-400 | 21 |
| 6 | 3 | <-400 | 26 | <-400 | 24 |
| 7 | 2 | <-400 | 19 | <-400 | 18 |
| 8 | 4 | <-400 | 25 | -100 | 12 |
| 9 | 3 | <-400 | 23 | <-400 | 26 |
| 10 | 2 | <-400 | 24 | <-400 | 23 |
| **Mean** | **2,9** | **-400** | **23** | **-370** | **21,6** |

The overall results show an unpredictably high efficiency of the invented device compared with the control group with the similar age and from the same operation waiting list.

### CONCLUSION OF THE STUDIES

The results above show an outstanding effect of the invented device compared with prior art. There are no former non-surgical inventions adapted to treat secretory otitis media in children below the age of 3. Clinical experience shows that patients older than 5-6 years might be able to use the devices presented in the prior art.

The new invention combines efficiency with comfort and compliance for treatment at home for children. The device shows not only an improved tympanometric result but also about 3 times better hearing in the treated subjects compared with the control group. No earlier known device has been able to show improved middle ear pressure on subjects as young as 1.5 year of age.

## Claims

1. Device for obtaining an equalization of a negative pressure in the middle ear of a human being, having a face with a nose and a mouth, the device comprising
a facemask (1), which facemask is adapted to tightly cover at least the nose and mouth, thereby creating an air chamber between the face and the facemask, and which facemask has a facemask air channel (2) which allows air flowing in or out of the chamber,
an air source (5), having the capacity to deliver an increased air pressure compared to the atmospheric air pressure through an air source outlet (13),
an air reservoir (7), having a reservoir air channel (8) which allows air flowing in or out of the reservoir,
and a tube structure (3), having at least three openings (14, 15, 16) adapted for connecting the facemask air channel (2), the air source outlet (13) and the reservoir air channel (8) to the tube structure interior, wherein the air reservoir (7) is a variable volume chamber like an elastic inflatable balloon or a variable volume chamber which is spring or gravity loaded and which air reservoir is designed to define and maintain an air pressure exceeding the atmosphere pressure and work like a pressure regulator when loaded, and
that the device is so adapted that when it is in use, an increased pressure is created by expiration into the facemask (1) and/ or activating the air source (5), thereby making the air reservoir (7) expand, creating increased pressure in the mouth/nose cavity and opening the Eustachian tube for air flow into the middle ear for equalization of the negative pressure, the air reservoir (7) then working as a pressure regulator.

2. A device according to claim 1, wherein the air reservoir (7) is designed to maintain a counter pressure of 200 - 800 daPa (20 - 80 mBar) when loaded.

3. A device according to any of the foregoing claims, wherein a security pressure relief valve (10) for pressure monitoring the tube structure interior is connected to the tube structure (3).

4. A device according to any of the foregoing claims, wherein an adjustable relief valve (11) is connected to the tube structure (3) to control the maximum pressure of the tube structure interior.

5. A device according to claim 4, wherein the adjustable relief valve (11) is adjustable to release pressure in the range from 0 to 100 mBar.

6. A device according to any of the foregoing claims, wherein a check valve (12) is connected between the air source (5) and the tube structure (3), allowing air flow into the tube structure.

7. A device according to claim 6, wherein the check valve (12) is arranged to cooperate with the adjustable relief valve (11) so that when the check valve is open the release valve is closed.

8. A device according to any of the foregoing claims, wherein the tube structure (3) comprises one or several interconnected tube shaped components, coupled to each other.

9. A device according to any of the foregoing claims, wherein the air source is a powered air pump (5), and that the air pump (5) comprises means for not permitting air flow in the opposite pump direction.

10. A device according to any of the claims 1-8, wherein the air source (5) is a manually operated bellow pump.

11. A device according to any of the foregoing claims, wherein the facemask (1) is removable from the connecting tube (3) in order to allow a choice of facemask of a size well suited for the human being in question to be provided.

12. A device according to any of the foregoing claims, wherein facemask (1) is provided with a piece (17) resembling a baby pacifier or a rubber nipple.

13. A device according to claim 12, wherein the piece (17) is replaceable and optional for installation in the facemask.

14. A device according to any of the foregoing claims, wherein the air reservoir (7) and/or the air source (5) is hidden in a funny figure in the shape of a puppet or a cuddle toy.

15. A device according to any of the foregoing claims, wherein the device comprises means for providing optical or visual feedback to the patient when the air reservoir is loaded.

## Patentansprüche

1. Vorrichtung zum Ausgleich eines Unterdrucks im Mittelohr eines Menschen, der ein Gesicht mit Nase und Mund hat, wobei die Vorrichtung folgendes umfasst:
eine Gesichtsmaske (1), die Gesichtsmaske mindestens Nase und Mund dicht bedeckt, wobei eine Luftkammer zwischen Gesicht und Gesichtsmaske gebildet wird und die Gesichtsmaske einen Gesichtsmasken-Luftkanal (2) aufweist, sodass Luft in die Kammer ein-, oder ausströmen kann,
eine Luftquelle (5), die einen im Vergleich zum atmosphärischen Luftdruck erhöhten Luftdruck durch einen Luftquellenauslass (13) liefern kann,
einen Luftbehälter (7) mit einem Behälterluftkanal (8), durch den Luft in den oder aus dem Behälter strömen kann, und
eine Rohrkonstruktion (3) mit mindestens drei Öffnungen (14, 15, 16), die so ausgelegt sind, dass sie den Gesichtsmasken-Luftkanal (2), den Luftquellenauslass (13) und den Behälterluftkanal (8) mit dem Inneren der Rohrkonstruktion verbinden, wobei
der Luftbehälter (7) eine Kammer mit veränderlichem Volumen ist, wie ein elastischer füllbarer Ballon oder eine Kammer mit veränderlichem Volumen, die feder- oder schwerkraftbefüllt ist, und die so ausgelegt ist, dass sie einen Luftdruck aufweist und aufrechterhält, der den Atmosphärendruck übersteigt, und im belasteten Zustand wie ein Druckregler wirkt und
dass die Vorrichtung so beschaffen ist, dass bei ihrer Verwendung durch Ausatmen in die Gesichtsmaske (1) und/oder Aktivieren der Luftquelle (5) ein erhöhter Druck erzeugt wird, wodurch sich der Luftbehälter (7) ausdehnt, was einen erhöhten Druck in der Mund/Nasenhöhle erzeugt und die Eustachische Röhre für einen Luftstrom in das Mittelohr zum Ausgleich des Unterdrucks öffnet, wobei der Luftbehälter (7) dann als Druckregler wirkt.

2. Vorrichtung nach Anspruch 1, wobei der Luftbehälter (7) so ausgelegt ist, dass er in gefülltem Zustand einen Gegendruck von 200 - 800 daPa (20 - 80 mBar) aufrechterhält.

3. Vorrichtung nach jedem der vorstehenden Ansprüche, wobei an die Rohrkonstruktion (3) ein Sicherheitsüberdruckventil (10) zur Drucküberwachung im Inneren der Rohrkonstruktion angeschlossen ist.

4. Vorrichtung nach jedem der vorstehenden Ansprüche, wobei ein einstellbares Überdruckventil (11) mit der Rohrkonstruktion (3) verbunden ist, um den maximalen Druck im Inneren der Rohrkonstruktion zu steuern.

5. Vorrichtung nach Anspruch 4, bei der das einstellbare Überdruckventil (11) zum Ablassen von Druck im Bereich von 0 bis 100 mBar einstellbar ist.

6. Vorrichtung nach jedem der vorhergehenden Ansprüche, wobei ein Rückschlagventil (12) zwischen der Luftquelle (5) und der Rohrkonstruktion (3) angeschlossen ist, das einen Luftstrom in die Rohrkonstruktion ermöglicht.

7. Vorrichtung nach Anspruch 6, wobei das Rückschlagventil (12) so angebracht ist, dass es mit dem einstellbaren Überdruckventil (11) zusammenwirkt, sodass das Überdruckventil geschlossen wird, wenn das Rückschlagventil geöffnet ist.

8. Vorrichtung nach jedem der vorangehenden Ansprüche, wobei die Rohrkonstruktion (3) eine oder mehrere miteinander verbundene rohrförmige Komponenten umfasst, die miteinander gekoppelt sind.

9. Vorrichtung nach jedem der vorangehenden Ansprüche, wobei die Luftquelle eine kraftbetriebene Luftpumpe (5) ist und die Luftpumpe (5) Einrichtungen umfasst, die einen Luftstrom in der entgegengesetzten Pumprichtung ausschließen.

10. Vorrichtung nach jedem der Ansprüche 1 - 8, wobei die Luftquelle (5) eine manuell betriebene Balgpumpe ist.

11. Vorrichtung nach jedem der vorangehenden Ansprüche, wobei die Gesichtsmaske (1) von dem Verbindungsrohr (3) abnehmbar ist, um eine Auswahl an Gesichtsmasken mit einer für den betreffenden Menschen geeigneten Größe zu ermöglichen.

12. Vorrichtung nach jedem der vorangehenden Ansprüche, wobei die Gesichtsmaske (1) mit einem Teil (17) versehen ist, das einem Säuglingsschnuller oder einem Gumminippel ähnel t.

13. Vorrichtung nach Anspruch 12, wobei das Teil (17) austauschbar ist und gegebenenfalls in die Gesichtsmaske (1) eingebaut werden kann.

14. Vorrichtung nach jedem der vorstehenden Ansprüche, wobei der Luftbehälter (7) und/oder die Luftquelle (5) in einer Spielfigur in Form einer Puppe oder eines Kuscheltiers verborgen ist.

15. Vorrichtung nach jedem der vorstehenden Ansprüche, wobei die Vorrichtung Einrichtungen umfasst, die dem Patienten eine optische oder visuelle Rückmeldung geben, wenn der Luftbehälter gefüllt ist.

## Revendications

1. Dispositif pour obtenir une égalisation d'une pression négative dans l'oreille moyenne d'un être humain ayant un visage avec un nez et une bouche, le dispositif comportant
un masque facial (1), lequel masque facial est adapté pour couvrir complètement au moins le nez et la bouche, créant ainsi un compartiment à air entre le visage et le masque facial, et lequel masque facial a un canal d'air de masque facial (2) qui permet à l'air de circuler dans le compartiment ou hors du compartiment,
une source d'air (5) ayant la capacité de fournir une augmentation de la pression d'air par rapport à la pression d'air atmosphérique à travers une sortie de source d'air (13),
un réservoir d'air (7) ayant un canal d'air de réservoir (8) qui permet à l'air de circuler dans le réservoir ou hors du réservoir, et
une structure tubulaire (3) ayant au moins trois ouvertures (14, 15, 16) adaptées pour connecter le canal d'air du masque facial (2), la sortie de source d'air (13) et le canal d'air du réservoir (8) à l'intérieur de la structure tubulaire, dans laquelle
le réservoir d'air (7) est un compartiment à volume variable comme un ballon élastique gonflable ou un compartiment à volume variable qui est chargé par ressort ou par gravité, lequel réservoir d'air est conçu pour définir et maintenir une pression d'air dépassant la pression atmosphérique, et fonctionner comme un régulateur de pression lorsqu'il est chargé, et
que le dispositif est adapté de telle sorte que, lorsqu'il est utilisé, une augmentation de la pression d'air est créée par l'expiration dans le masque facial (1) et/ou l'activation de la source d'air (5), amenant ainsi le réservoir d'air (7) à se déployer, et cela crée une plus grande pression dans la cavité buccale/nasale et ouvre la trompe d'Eustache pour permettre à l'air de circuler dans l'oreille moyenne afin d'égaliser la pression négative, le réservoir d'air (7) fonctionnant alors comme un régulateur de pression.

2. Dispositif selon la revendication 1, dans lequel le réservoir d'air (7) est conçu pour maintenir une contrepression de 200 - 800 daPa (20 - 80 mBar) lorsqu'il est chargé.

3. Dispositif selon l'une quelconque des revendications précédentes, dans lequel une soupape de sécurité (10) destinée à contrôler la pression à l'intérieur de la structure tubulaire est reliée à la structure tubulaire (3) .

4. Dispositif selon l'une quelconque des revendications précédentes, dans lequel une soupape de décharge réglable (11) est reliée à la structure tubulaire (3) pour contrôler la pression maximale de l'intérieur de la structure tubulaire.

5. Dispositif selon la revendication 4, dans lequel la soupape de décharge réglable (11) est réglable pour relâcher la pression dans la plage de 0 à 100 mBar.

6. Dispositif selon l'une quelconque des revendications précédentes, dans lequel un clapet anti-retour (12) est relié entre la source d'air (5) et la structure tubulaire (3), permettant à l'air de circuler dans la structure tubulaire.

7. Dispositif selon la revendication 6, dans lequel le clapet anti-retour (12) est disposé de manière à coopérer avec la soupape de décharge réglable (11) de sorte que lorsque le clapet anti-retour est ouvert, la soupape de décharge est fermée.

8. Dispositif selon l'une quelconque des revendications précédentes, dans lequel la structure tubulaire (3) comprend un ou plusieurs composants en forme de tube interconnectés, reliés les uns aux autres.

9. Dispositif selon l'une quelconque des revendications précédentes, dans lequel la source d'air est une pompe à air motorisée (5), et la pompe à air (5) comprend des moyens pour ne pas permettre à l'air de circuler dans la direction opposée de la pompe.

10. Dispositif selon l'une quelconque des revendications 1 à 8, dans lequel la source d'air (5) est une pompe à soufflet à commande manuelle.

11. Dispositif selon l'une quelconque des revendications précédentes, dans lequel le masque facial (1) est détachable du tube de connexion (3) afin de permettre un choix de masque facial de taille bien adaptée à l'être humain en question.

12. Dispositif selon l'une quelconque des revendications précédentes, dans lequel le masque facial (1) est muni d'une pièce (17) ressemblant à une sucette pour bébé ou à une tétine en caoutchouc.

13. Dispositif selon la revendication 12, dans lequel la pièce (17) est remplaçable et optionnelle pour une mise en place dans le masque facial (1).

14. Dispositif selon l'une quelconque des revendications précédentes, dans lequel le réservoir d'air (7) et/ou la source d'air (5) est caché(e) dans une figurine amusante en forme de marionnette ou de peluche.

15. Dispositif selon l'une quelconque des revendications précédentes, dans lequel le dispositif comprend des moyens pour fournir un retour optique ou visuel au patient lorsque le réservoir d'air est chargé.
